# EUROPEAN PATENT SPECIFICATION

(11) **EP 2 015 689 B1**
(45) Date of publication and mention of the grant of the patent: **03.11.2010**
(21) Application number: 07732720.3
(22) Date of filing: 08.05.2007
(51) Int. Cl.: A61B 17/50

(54) **DEVICE FOR REMOVING AN EXTERNAL PARASITE**
VORRICHTUNG ZUR ENTFERNUNG EINES EXTERNEN PARASITEN
DISPOSITIF DESTINÉ À ENLEVER UN PARASITE EXTERNE

(30) Priority: 09.05.2006 GB 0609157
(43) Date of publication of application: 21.01.2009
(73) Proprietor: Pabari, Alpa Shantilal, Chelmsford, Essex CM2 6AR (GB); Pabari, Hiten Shantilal, Chelmsford, Essex CM2 6AR (GB)
(72) Inventor: Pabari, Alpa Shantilal, Chelmsford, Essex CM2 6AR (GB); Pabari, Hiten Shantilal, Chelmsford, Essex CM2 6AR (GB)
(74) Representative: Want, Clifford James
(86) International application number: PCT/GB2007/001692
(87) International publication number: WO 2007/129095

(56) References cited:
- WO-A-03/022094
- WO-A-2004/054457
- DE-U1- 29 722 310
- US-A- 5 447 511

## Description

This invention relates to a device for removing an external parasite from the epidermis and dermis of a host and, in particular, to a tick remover.

Various shapes and designs of tools for removing ticks and similar parasites from the skin of humans and animals are known.

Although tweezers and similar pinching devices may be used for tick removal, there is a tendency to engage nearby skin or hair of the host in a painful manner. In addition, there is a tendency to squeeze the tick which may release bacteria from the tick into the bloodstream of the host. Moreover, the tick body may rupture leaving a portion of the body in or beneath the skin of the host. In addition, it is desirable to remove a tick in a rapid operation that does not involve preliminary manipulation so as not to provide a stimulus and sufficient time for the tick to disgorge or vomit, which may also release bacteria to the host.

Preferably, a single tool should be suitable for removing different sizes of parasite.

US 5,447,511 discloses a tick removal tool comprising an elongate blade member having a tapered first end in which there is provided a single V-shaped slot having a major axis along a longitudinal axis of the elongate member. Opposed arms defining the V-shaped slot are rounded at their outer extremities. A lower face of the elongate blade member is apparently transversely downwardly concave and the arms defining the V-shaped slot appear to be upwardly arcuate. A tick engaged within the V-shaped slot is either lifted upwards away from the skin of the host or the tool is pivoted against the skin to pry the tick from the skin.

US 5,595,569 discloses a spoon-shaped device for removing ticks from pets and other hosts in which an outer or leading edge of the spoon bowl has a single bevelled V-shaped notch, so that a portion of the bowl may be passed between a tick and the skin of the host until the scoop engages a body of the tick on three sides. The device then has to be slid forward to release the tick.

US 5,607,434 discloses an elongate tick-removing device having an upwardly arcuate tick engaging end having a V-shaped notch having a longitudinal axis on the longitudinal axis of the device. A tick is removed by sliding the V-shaped notch under the tick to lift the tick away from the flesh. Either a scoop or an adhesive patch are provided at an inner end of the V-shaped notch to retain a removed tick.

US 5,876,409 discloses a generally hook shaped implement for removing ticks in which a shank end of the hook shape forms a handle and an opposed end has a V-shaped notch or claw with chamfered edges. In use opposed prongs of the claw are passed on respective opposed sides of the tick and the tick removed by rotating the implement about a longitudinal axis through the handle portion while pulling slightly away from the skin, so that the tick is neither merely lifted or squeezed but removed essentially by rotation.

US 6,102,409 discloses a generally arcuate L-shaped or swan-neck shaped implement for removing parasites such as ticks, the implement including a handle portion, a fulcrum portion and a forked portion. Ticks are removed by rotating the implement about the arcuate fulcrum portion in a manner of a claw hammer removing a nail. The apparatus includes a spoon-like portion for retaining a tick after removal.

US D496,460 illustrates an elongate tick remover apparently comprising a chamfered V-shaped notch having a longitudinal axis aligned with a longitudinal axis of an elongate member.

WO 2004/054457 discloses a tool for removing ticks and like parasites from the skin of a host using a V-shaped groove having concave or stepped opposing inner faces to provide space within the notch for receiving the head and/or body of a tick without the tick being squeezed between the opposing engagement faces of the V-shaped groove. In one embodiment the tool compromises an elongate member with an axis of the V-shaped groove aligned with a longitudinal axis of the elongate member. In use the tool is flexed with an engagement portion including the V-shaped groove substantially parallel to, and in contact with, the skin containing a tick to be removed, and a handle portion at an acute angle to the skin. The tool is thus moved towards and past the position of the tick, thereby engaging the tick in the V-shaped groove and removing the tick from the skin. In an alternative embodiment the tool is of the shape and size of a credit card, for storing with credit cards, and having the V-shaped grooved in one apex thereof with an axis at approximately 45° to major axes of the tool. Such engagement grooves may be provided in two neighbouring apexes to permit use by a right hand or a left hand. Finger holds are provided in the edges of the credit card shaped tool to provide purchase on the tool.

DE 297 22 310 U1 discloses a device according to the preamble of claim 1.

The invention is defined by claim 1.

Preferably, the projection is angled at an acute angle with respect to the substantially rectangular plate in a direction away from a plane defined by the substantially rectangular plate and towards the upper face.

Conveniently, the projection is arcuate in a direction perpendicular to the plane defined by the substantially rectangular plate.

Advantageously, the device further comprises a second V-shaped, chamfered notch, smaller than first V-shaped notch, in an edge of the substantially rectangular plate opposed to the edge on which the projection is located.

Advantageously, the second V-shaped notch is located at a first apex of the substantially rectangular plate and a third V-shaped notch is located at a second apex in the edge of the substantially rectangular plate opposed to the edge on which the projection is located.

Conveniently, the third V-shaped notch is smaller than the second V-shaped notch.

Preferably, the device further comprises a first indentation in an upper face of the substantially rectangular plate adapted to provide additional purchase to a tip of a thumb of a user.

Conveniently, the device further comprises a second indentation in an lower face of the substantially rectangular plate adapted to provide additional purchase to a fingertip of a user.

The invention will now be described, by way of example, with reference to the accompanying drawings in which:
Figure 1 is a top view of a tick remover according to the invention;
Figure 2 is a side view of the tick remover of Figure 1; and
Figure 3 is an end view of the tick remover of Figure 1.

In the Figures, like reference numbers denote like parts.

Referring to the Figures, a tick remover 10, according to the invention, comprises a substantially rectangular plate 11, preferably of a resilient material. An engagement minor end of the substantially rectangular plate has rounded convex apexes 121 and is provided therebetween with a projection 13 incorporating an outwardly facing first V-shaped notch 131 having a longitudinal axis aligned with a longitudinal axis of the substantially rectangular plate 11, so that the projection is divided by the first V-shaped notch into two prongs 134, 135.

A suitable overall size of the tick remover is 70 mm long and 40 mm wide, with a rectilinear portion of the substantially rectangular plate 11 having a length of 31 mm.

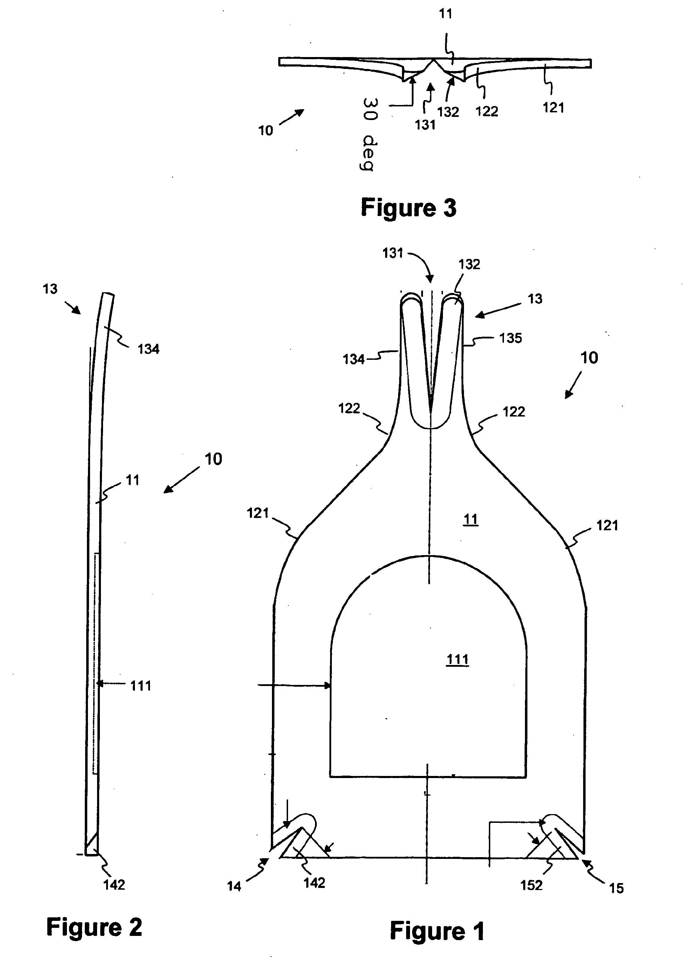

Opposing inner engagement faces of the two prongs 134, 135 are provided with a upward facing chamfer 132. Tips of the prongs 134, 135 are rounded. Outer portions of outer edges of the prongs 134, 135 are substantially linear and mutually parallel and have concave transitional portions 122 to the convex apexes 121 of the substantially rectangular plate 11. As best seen in Figure 2, the prongs 134, 135 are angled arcuately upwards with respect to a plane defined by the substantially rectangular plate 11.

A suitable length of the V-shaped notch is 15 mm and a suitable width of opening of 3 mm. A suitable width of the projection 13 at an outer end thereof is 8 mm. A suitable angle of the chamfer is 30°.

Apexes of the substantially rectangular plate 11 opposed to the convex apexes 12 are provided with a second V-shaped notch 14 and a third V-shaped notch 15 respectively, both smaller than the first V-shaped notch 131, and having axes inclined respectively at substantially 45° to the longitudinal axis of the substantially rectangular plate 11, thereby intersecting the rectangular apexes of the substantially rectangular plate 11 opposed to the rounded apexes 12. Conveniently, an opening of the second V-shaped notch 14 is substantially a half of the width of the opening of the first V-shaped notch 131. Conveniently an opening of the third V-shaped notch 15 is smaller than the opening of the second V-shaped notch 14, and conveniently a width of the opening of the third V-shaped notch 15 is two thirds of a width of an opening of the second V-shaped notch 14. The second and third V-shaped notches 14, 15 are provided with chamfers 142, 152 respectively, similar to the chamfer 132 of the first V-shaped notch 131.

A suitable length of the second and third V-shaped notches 14, 15 is 5 mm respectively, a suitable opening of the second V-shaped notch is 1.5 mm and a suitable opening of the third V-shaped notch is 1 mm.

An upper surface of the substantially rectangular plate 11 is provided with a, generally arch-shaped, indentation 111 having a rectangular portion remote from the engagement minor end of the substantially rectangular plate 11 and a semicircular portion proximate the engagement minor end. The indentation is sized to receive a thumb tip of a user. A suitable width of the indentation is 25 mm and a suitable overall length 17.5 mm. An opposed lower surface of the substantially rectangular plate 11 may be provided with a corresponding indentation, not shown, to receive a tip of an opposing finger, such as a forefinger, of a user.

An aperture, not shown, may be provided in the substantially rectangular plate 11, located for example substantially between the second and third V-shaped notches, for receiving, for example, a ring, chain or thong by which the tick remover may be retained or suspended.

In use, the prongs of the tick remover are slid, with the prongs parallel to, and in contact with, the skin of a host and the substantially rectangular plate 11 angled to the skin, towards a tick or other external parasite so that a respective prong passes on each side respectively of the tick or other parasite to be removed from the skin of the host so that the parasite is engaged by the inner opposed engagement sides of the prongs and immediately removed from the skin. It will be understood that alternatively, or in addition, once a parasite is engaged by the opposed engagement faces, the tick remover may be rotated to twist the parasite to assist removal from the skin.

The second V-shaped notch may similarly be used for smaller parasites than can be grasped with the first V-shaped notch and the third V-shaped notch may be used for smaller parasites than can be grasped with the second V-shaped notch.

Providing notches of different sizes provides an advantage that an external parasite attached to the skin of a host can be engaged by an appropriately sized notch sufficiently small to engage a portion of the parasite closest to the skin, while sufficiently large to minimise a danger of the engagement faces squeezing or squashing the parasite. This provision of multiple sizes of notch thus obviates any requirement for complex shaped engagement faces of the notches such as faces which are concave or stepped in cross-section.

A tip of a thumb of a user may be inserted in the indentation in the upper face to provide additional purchase on the plate to assist in rapidly removing the parasite from the skin. Optionally a tip of an opposed forefinger may similarly be inserted in the opposed indentation in the lower face. It will be understood that in any case the width of the substantially rectangular plate provides improved purchase on the tick remover compared with narrower tools of the prior art, while the relative narrowness of the projection containing the first V-shaped notch tends to avoid hairs of the skin becoming entrapped in, or entangled with, the notch or other parts of the tool. This avoidance of entanglement is added by the arcuate concave-convex transition from sides of the projection to sides of the substantially rectangular plate, which tends to sweep hairs aside. It will be further understood that alignment of the first V-shaped notch with an axis of the substantially rectangular plate, and holding the tool substantially on the longitudinal axis, substantially avoids the production of any moment tending to cause the device to rotate upon engagement of the V-shaped notch with a parasite. Hence any necessity for finger holds in the sides of a plate to resist such rotation is thus avoided.

Although the invention has been described with a projection central of a minor edge of the substantially rectangular plate, it will be understood that alternately the projection could be located central of a major edge.

## Claims

1. A device (10) for removing an external parasite from a host, the device comprising a substantially rectangular plate (11) having a projection (13) from a minor or major edge thereof, the projection comprising two prongs (134, 135) defining a first inward tapering V-shaped notch (131) therebetween having a longitudinal axis aligned with an axis of the substantially rectangular plate and having opposed inner chamfered engagement faces (132) for engaging substantially opposed sides respectively of the external parasite, **characterised by** the projection being of a width less than a quarter of a length of the edge and by an arcuate concave-convex transition (121, 122) from sides of the projection (13) to sides of the substantially rectangular plate (11).

2. A device as claimed in claim 1, wherein the projection (13) is angled at an acute angle with respect to the substantially rectangular plate (11) in a direction away from a plane defined by the substantially rectangular plate and towards the upper face.

3. A device as claimed in claim 2, wherein the projection (13) is arcuate in a direction perpendicular to the plane defined by the substantially rectangular plate (11).

4. A device as claimed in any of the preceding claims, further comprising a second V-shaped, chamfered notch (14, 15), smaller than first V-shaped notch (131), in an edge of the substantially rectangular plate (11) opposed to the edge on which the projection is located.

5. A device as claimed in claim 4, wherein the second V-shaped notch (14) is located at a first apex of the substantially rectangular plate and a third V-shaped notch (15) is located at a second apex in the edge of the substantially rectangular plate opposed to the edge on which the projection (13) is located.

6. A device as claimed in claim 5, wherein the third V-shaped notch (15) is smaller than the second V-shaped notch (14).

7. A device as claimed in any of the preceding claims, further comprising a first indentation (111) in an upper face of the substantially rectangular plate (11) adapted to provide additional purchase to a tip of a thumb of a user.

8. A device as claimed in any of the preceding claims, further comprising a second indentation in a lower face of the substantially rectangular plate (11) adapted to provide additional purchase to a fingertip of a user.

## Patentansprüche

1. Vorrichtung (10) zum Entfernen eines externen Parasiten von einem Wirt, wobei die Vorrichtung eine im Wesentlichen rechteckige Platte (11) mit einem Vorsprung (13) von einer Neben- oder Hauptkante der Platte umfasst, wobei der Vorsprung zwei Zinken (134, 135) umfasst, die dazwischen eine erste, einwärts konisch verlaufende V-förmige Kerbe (131) mit einer Längsachse definieren, die mit einer Achse der im Wesentlichen rechteckigen Platte ausgerichtet ist und gegenüberliegende innere, abgeschrägte Eingriffsflächen (132) für einen Eingriff mit entsprechenden im Wesentlichen gegenüberliegenden Seiten des externen Parasiten aufweist, **dadurch gekennzeichnet, dass** der Vorsprung eine Breite aufweist, die kleiner ist als ein Viertel einer Länge der Kante und mit einem bogenförmigen konkavkonvexen Übergang (121, 122) von den Seiten des Vorsprungs (13) zu den Seiten der im Wesentlichen rechteckigen Platte (11).

2. Vorrichtung nach Anspruch 1, wobei der Vorsprung (13) in einem spitzen Winkel im Verhältnis zu der im Wesentlichen rechteckigen Platte (11) in eine von einer durch die im Wesentlichen rechteckige Platte definierten Ebene weggehenden Richtung angewinkelt ist, sowie in Richtung der oberen Seite.

3. Vorrichtung nach Anspruch 2, wobei der Vorsprung (13) bogenförmig ist in eine Richtung, die senkrecht zu der Ebene ist, die durch die im Wesentlichen rechteckige Platte (11) definiert wird.

4. Vorrichtung nach einem der vorstehenden Anspruche, wobei diese ferner eine zweite V-förmige, abgeschrägte Kerbe (14, 15) umfasst, die kleiner ist als die erste V-förmige Kerbe (131), in einer Kante der im Wesentlichen rechteckigen Platte (11) gegenüber der Kanate, an der sich der Vorsprung befindet.

5. Vorrichtung nach Anspruch 4, wobei sich die zweite V-förmige Kerbe (14) an einem ersten Scheitel der im Wesentlichen rechteckigen Platte befindet, und wobei eine dritte V-förmige Kerbe (15) an einem zweiten Scheitel in der Kante der im Wesentlichen rechteckigen Platte angeordnet ist, die gegenüber der Kante angeordnet ist, an der sich der Vorsprung (13) befindet.

6. Vorrichtung nach Anspruch 5, wobei die dritte V-förmige Kerbe (15) kleiner ist als die zweite V-förmige Kerbe (14).

7. Vorrichtung nach einem der vorstehenden Ansprüche, wobei diese ferner eine erste Vertiefung (111) in einer oberen Seite der im Wesentlichen rechteckigen Platte (11) umfasst, geeignet um für eine Spitze eines Daumens eines Anwenders zusätzlichen Halt bereitzustellen.

8. Vorrichtung nach einem der vorstehenden Ansprüche, wobei diese ferner eine zweite Vertiefung in einer unteren Seite der im Wesentlichen rechteckigen Platte (11) umfasst, geeignet um für eine Fingerspitze eines Anwenders zusätzlichen Haft bereitzustellen.

## Revendications

1. Dispositif (10) destiné à enlever un parasite externe d'un hôte, le dispositif comprenant une plaque sensiblement rectangulaire (11) ayant une projection (13) depuis un bord mineur ou majeur de celle-ci, la projection comportant deux griffes (134, 135) définissant une première encoche en forme de V conique vers l'intérieur (131) entre elles ayant un axe longitudinal aligné avec un axe de la plaque sensiblement rectangulaire et ayant des faces de mise en prise chanfreinées intérieures opposées (132) pour mettre en prise des côtés sensiblement opposés respectivement du parasite externe, **caractérisé en ce que** la projection est d'une largeur inférieure à un quart de la longueur du bord et par une transition concave-convexe arquée (121, 122) depuis des côtés de la projection (13) jusqu'à des côtés de la plaque sensiblement rectangulaire (11).

2. Dispositif selon la revendication 1, dans lequel la projection (13) est inclinée à un angle aigu par rapport à la plaque sensiblement rectangulaire (11) dans une direction opposée à un plan défini par la plaque sensiblement rectangulaire et vers la face supérieure.

3. Dispositif selon la revendication 2, dans lequel la projection (13) est arquée dans une direction perpendiculaire au plan défini par la plaque sensiblement rectangulaire (11).

4. Dispositif selon l'une quelconque des revendications précédentes, comprenant en outre une deuxième encoche chanfreinés en forme de V (14, 15), plus petite que la première encoche en forme de V (131), dans un bord de la plaque sensiblement rectangulaire (11) opposé au bord sur lequel la projection est située.

5. Dispositif selon la revendication 4, dans lequel la deuxième encoche en forme de V (14) est située au niveau d'un premier sommet de la plaque sensiblement rectangulaire et une troisième encoche en forme de V (15) est située au niveau d'un second sommet dans le bord de la plaque sensiblement rectangulaire opposé au bord sur lequel la projection (13) est située.

6. Dispositif selon la revendication 5, dans lequel la troisième encoche en forme de V (15) est plus petite que la deuxième encoche en forme de V (14).

7. Dispositif selon l'une quelconque des revendications précédentes, comprenant en outre une première indentation (111) dans une face supérieure de la plaque sensiblement rectangulaire (11) adaptée pour fournir un point d'appui supplémentaire au bout d'un pouce d'un utilisateur.

8. Dispositif selon l'une quelconque des revendications précédentes, comprenant en outre une seconde indentation (111) dans une face inférieure de la plaque sensiblement rectangulaire (11) adaptée pour fournir un point d'appui supplémentaire au bout d'un doigt d'un utilisateur.
